# EUROPEAN PATENT SPECIFICATION

(11) **EP 1 112 038 B1**
(45) Date of publication and mention of the grant of the patent: **12.11.2003**
(21) Application number: 99946885.3
(22) Date of filing: 10.09.1999
(51) Int. Cl.: A61F 2/00

(54) **PREFORMED CURVED PROSTHESIS HAVING A REDUCED INCIDENCE OF DEVELOPING WRINKLES OR FOLDS**
VORGEFORMTE GEKRÜMMTE PROTHESE MIT EINEM VERMINDERTEN AUFTRETEN VON FALTEN
PROTHESE INCURVEE PREFORMEE PRESENTANT UNE SUSCEPTIBILITE LIMITEE A L'APPARITION DE PLIS

(30) Priority: 11.09.1998 FR 9811331
(43) Date of publication of application: 04.07.2001
(73) Proprietor: C.R. Bard Inc., Murray Hill, NJ 07974 (US)
(72) Inventor: PAJOTIN, Philippe, F-49300 Cholet (FR)
(74) Representative: Grey, Ian Michael
(86) International application number: US9920933
(87) International publication number: WO00015142

(56) References cited:
- EP-A- 0 836 838
- WO-A-95/07666
- US-A- 3 559 214
- US-A- 5 236 454
- US-A- 5 584 884

## Description

### Background of the Invention

### 1. Field of the Invention

The present invention relates to an implantable prosthesis, and more particularly to a prosthesis for repairing a hernia.

### 2. Description of the Related Art

A defect in a muscle or tissue wall, such as a hernia, is commonly repaired with an implantable prosthesis that is configured to cover and/or fill the defect. In many instances, a flat sheet of an implantable, non-resorbable, flexible mesh material, such as BARD MESH, has been employed for the parietal repair of hernias and eventrations of the abdominal wall. However, a surgeon may experience some difficulty positioning the mesh between the parietal peritoneum and the abdominopelvic wall. The mesh may also fold or wrinkle and be difficult to maintain in position.

Applicant previously developed an implantable prosthesis for repairing a defect in a muscle or tissue wall to alleviate some of these concerns. The prosthesis, which is disclosed in WO 95/07666 and is assigned to C.R. Bard, the assignee of the present application, is made of an implantable, nonabsorbable and flexible material that is formed to independently assume a curved shape adapted to conform to the anatomical shape of the wall. The prosthesis includes a body comprised of a first portion having a substantially spherical shape and a second portion connected to an outer edge of the first portion.

This prosthesis has proven useful and has become established in the practice of muscle or tissue wall repair in the inguinofemoral region. The prosthesis is not subject to stresses when deformed and, therefore, has no tendency to shift upon implantation.

It has nevertheless been observed that the prosthesis at times may wrinkle or fold between the first and second portions, thereby reducing the overall size of the prosthesis which may result in a prosthesis that does not adequately cover the region being repaired.

It is an object of the present invention to provide an improved prosthesis for repairing a defect in a muscle or tissue wall.

### Summary of the Invention

The present invention is an implantable prosthesis for repairing a defect in a muscle or tissue wall. The prosthesis includes a body of prosthetic material having a preformed three-dimensional contoured shape that independently assumes a curved shape adapted to conform to the wall. The body includes a first portion that is configured with a substantially spherical shape and a second portion that is connected to the first portion at an outer edge thereof, wherein the second portion has a shape that is distinct from the first portion. The first portion has a first radius of curvature and the second portion has a second radius of curvature that is substantially equal to the first radius of curvature along the outer edge. The body is configured to substantially reduce the incidence of wrinkles or folds between the first and second portions so that the first and second portions do not partially cover each other upon or after implantation, thereby ensuring that the overall size of the prosthesis is sufficient to adequately cover the desired portion of the wall.

According to another preferred feature of the invention, the first portion includes a first lower edge and the second portion includes a second lower edge, and the body further includes a third portion connected to the first lower edge and a fourth portion connected to the second lower edge. Each of the third and fourth portions is configured with a substantially spherical shape to enhance conformance to a particular anatomical shape.

According to a further preferred feature of the invention, the prosthesis has a total surface area greater than approximately 40,000 mm².

According to still another preferred feature of the invention, the prosthesis is configured with a ratio of the surface area of the second portion to the total surface area of the prosthesis from approximately 0.25 to approximately 0.40.

Further preferred features are defined in dependent claims.

### Brief Description of the Drawings

The foregoing and other objects and advantages of the invention will be appreciated more fully from the following drawings, given solely by way of example, in which:
Figure 1 is a plan view of a prosthesis according to one illustrative embodiment of the invention;
Figure 2 is a cross-sectional view of the prosthesis taken along section line II-II in Figure 1; and
Figure 3 is a cross-sectional view of the prosthesis taken along section line III-III in Figure 1.

### Detailed Description

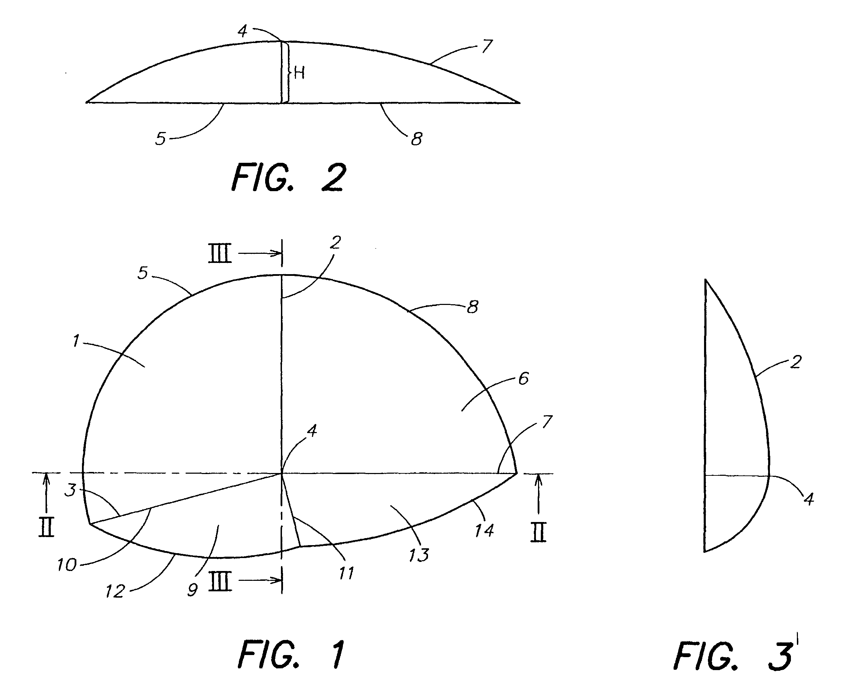

FIGS. 1-3 illustrate one embodiment of a prosthesis for repairing a defect in a muscle or tissue wall, such as a hernia. The prosthesis includes a body of prosthetic material that is preformed with a three-dimensional configuration that conforms to the anatomical shape of the defective wall to facilitate placement and minimize shifting of the prosthesis when positioned on the wall. The body includes a plurality of shaped portions that are joined to each other to create a desired configuration.

In one illustrative embodiment, the body includes first, second, third and fourth portions 1, 6, 9, 13, respectively, that are connected to each other to form a unitary structure. Each of the portions includes a curved surface that provide the prosthesis with the preformed contoured shape.

The first portion 1 is formed with a substantially spherical shape and includes a first outer edge 2 and a first lower edge 3 joined to each other at an apex 4 of the prosthesis, which is the highest point thereof. The first portion 1 also includes a circular inner edge 5 that extends in the same plane as the outer and lower edges 2, 3.

The second portion 6, which lies adjacent the first portion 1, includes a second lower edge 7 and a circular second outer edge 8. The second portion 6 is connected to the first portion 1 along the first outer edge 2.

The third portion 9 is disposed below the first portion 1 and includes an upper edge 10, a third outer edge 11 and a curved third lower edge 12. As shown, the upper edge 10 merges with the first lower edge 3 of the first portion.

The fourth portion 13 is positioned below the second portion 6 and adjacent the third portion 9. The fourth portion 13 is defined by and is connected to the second lower edge 7 of the second portion 6 and the third outer edge 11 of the third portion 9. The fourth portion also includes a curved fourth lower edge 14.

As illustrated in FIG. 1, the inner edge 5, the second outer edge 8 and the third and fourth lower edges 12, 14 form a generally D-shaped peripheral edge of the prosthesis. The peripheral edge may be welded or fused so that the body can regain its contoured shape after being deformed during implantation.

Each of the third and fourth portions 9, 13 may also have substantially spherical shapes to enhance conformance to a particular anatomical shape. In one illustrative embodiment, the radius of curvature of the third and fourth portions is less than the radius of curvature of the first portion to form surfaces in the third and fourth portions that have a steeper incline relative to the first portion.

The third and fourth portions are shaped to form a depression in the surface of the body that is configured to receive the iliac vessels when the prosthesis is employed for inguinal hernia repair. The depression extends inwardly from the peripheral edge between the third and fourth lower edges 12, 14 toward the apex 4.

The prosthesis may be formed from an implantable, biologically compatible material that is nonabsorbable and flexible. In one embodiment, the prosthesis is formed of a knitted fabric of implantable polypropylene filament, such as BARD MESH, that includes a plurality of interstices (not shown) for promoting tissue ingrowth to the prosthesis. It is to be understood, however, that the prosthesis may be formed of any suitable material.

The prosthesis may be configured to have any size suitable for a particular application. In one embodiment, the height H of the prosthesis from a plane defined by the peripheral edge and the apex 4 is approximately 21 mm. The first portion 1 has a substantially spherical shape with a radius of curvature of approximately 120 mm, particularly along the first outer edge 2. The second portion 6 has substantially the same radius of curvature of approximately 120 mm adjacent the first outer edge 2. The third and fourth portions 9, 13 each has a substantially spherical shape with a radius of curvature of approximately 35 mm. The total surface area of the prosthesis is approximately 44,780 mm², with the second portion 10 having a surface area of approximately 12,735 mm².

The disclosed configuration is particularly suited for repairing an inguinal hernia. It is to be appreciated, however, that this configuration is exemplary and that the prosthesis may be configured to have other sizes suitable for a particular application.

The preformed curved shape of the prosthesis may be obtained using a thermoforming procedure. In one illustrative embodiment, the thermoforming procedure includes placing a sheet of mesh fabric in a mold having the desired shape for the prosthesis, heating the fabric in the mold at an approximate temperature of 100°C to 200°C for a period of approximately 5 to 60 minutes, and subsequently cooling the fabric in the mold with an air flow having an approximate temperature of 15°C to 30°C for a period of approximately 5 to 60 minutes.

The edges of the prosthesis may be welded by fusing the meshes and the material using an ultrasonic welding procedure. During this procedure, the prosthesis is maintained between an element generating vibrations and an anvil that is configured to the particular dimensions of the prosthesis. In one embodiment, the edges are welded at a pressure of approximately 150 kPa to 800 kPa and an energy of approximately 100 to 5000 joules for a period of approximately 50 to 5000 milliseconds.

Once the sheet of mesh fabric has been shaped and the edges of the shaped prosthesis have been welded, any excess fabric extending beyond the welded edges is separated from the body of the prosthesis using a manual cutting procedure to form the completed prosthesis.

After inspection, the prosthesis may be packed in an internal packing (shell and insert) that has been designed specifically according to the three-dimensional characteristics of the prosthesis so as to comply with and protect the preformed curved shape of the prosthesis. The internal packing may be subsequently placed and packaged in a external packing for additional protection. The entire assembly may then be sterilized using any suitable method, such as with ethylene oxide, to provide a sterile prosthesis that is ready for implantation.

It should be understood that the foregoing description of the invention is intended merely to be illustrative thereof and that other embodiments, modifications, and equivalents of the invention are within the scope of the invention recited in the claims appended hereto.

## Claims

1. An implantable prosthesis for repairing a defect in a muscle or tissue wall, the prosthesis comprising:
a body of prosthetic material having a preformed three-dimensional contoured shape that independently assumes a curved shape adapted to conform to the wall, the body including a first portion (1) configured with a substantially spherical shape and a second portion (6) connected to the first portion at an outer edge (2) thereof, wherein the second portion has a shape that is distinct from the first portion, the first portion having a first radius of curvature, **characterized in that** the second portion has a second radius of curvature that is substantially equal to the first radius of curvature along the outer edge (2) in a direction perpendicular to the outer edge (2).

2. The prosthesis according to claim 1, wherein the second portion terminates in an end opposite the outer edge and has a curved shape extending from the outer edge to the end in a direction perpendicular to the outer edge (2).

3. The prosthesis according to claim 2, wherein the end of the second portion includes a pointed tip.

4. The prosthesis according to one of claims 2 to 3, wherein the second radius of curvature differs from the first radius of curvature along a segment of the second portion between the outer edge (2) and the end thereof.

5. The prosthesis according to any of the preceding claims, wherein the first portion (1) includes a first lower edge (3) and the second portion (6) includes a second lower edge (7), and wherein the body includes a third portion (9) connected to the first lower edge (3) and a fourth portion (13) connected to the second lower edge (7), each of the third and fourth portions configured with a substantially spherical shape.

6. The prosthesis according to any of the preceding claims, wherein the second portion has a surface area and the body has a total surface area, the prosthesis being configured with a ratio of the surface area of the second portion to the total surface area that ranges from approximately 0.25 to approximately 0.40.

7. The prosthesis according to claim 6, wherein the total surface area is greater than approximately 40,000 mm².

8. The prosthesis according to one of claims 5 to 7, wherein the body includes a permanent depression between the third portion (9) and the fourth portion (13), the depression being constructed and arranged to be placed proximate the external iliac vessels when the prosthesis is positioned on the wall to repair an inguinal hernia.

9. The prosthesis according to claim 8, wherein the body includes an apex (4), the depression extending inwardly from a peripheral edge of the body to ward the apex.

10. The prosthesis according to one of claims 5 to 9, wherein the third portion (9) has a third radius of curvature and the fourth portion (13) has a fourth radius of curvature that is substantially equal to the third radius of curvature.

11. The prosthesis according to claim 10, wherein the third radius of curvature is approximately 35mm.

12. The prosthesis according to one of claims 5 to 11, each of the third and fourth portions (9, 13) includes a curved lower edge (12, 14).

13. The prosthesis according to any of the preceding claims, wherein the body includes a peripheral edge that is generally D-shaped.

14. The prosthesis according to any of the preceding claims, wherein the first radius of curvature is approximately 120mm.

15. The prosthesis according to any of the preceding claims, wherein the body of prosthetic material includes surgical mesh.

16. The prosthesis according to any of the preceding claims, wherein the body includes a peripheral edge that is configured to allow the body to regain the contoured shape after being deformed.

## Patentansprüche

1. Implantierbare Prothese zum Reparieren eines Defekts in einer Muskel- oder Gewebewand, wobei die Prothese umfasst:
einen Körper aus prothetischem Material, der eine vorgeformte dreidimensional konturierte Gestalt aufweist, die selbständig eine gekrümmte Gestalt annimmt, welche ausgebildet ist, um sich an die Wand anzupassen, wobei der Körper einen ersten Abschnitt (1 umfasst, der in einer im Wesentlichen sphärischen Gestalt konfiguriert ist, und einen zweiten Abschnitt (6), der mit dem ersten Abschnitt an seinem Außenrand (2) verbunden ist, wobei der zweite Abschnitt eine Gestalt aufweist, die sich vom ersten Abschnitt unterscheidet und der erste Abschnitt einen ersten Krümmungsradius aufweist, **dadurch gekennzeichnet, dass** der zweite Abschnitt einen zweiten Krümmungsradius aufweist, der im Wesentlichen gleich dem ersten Krümmungsradius längs des Außenrandes (2) in Richtung senkrecht zum Außenrand (2) ist.

2. Prothese nach Anspruch 1, wobei der zweite Abschnitt in einem dem Außenrand entgegengesetzten Ende ausläuft und eine gekrümmte Gestalt aufweist, welche sich vom Außenrand in Richtung senkrecht zum Außenrand (2) zum Ende hin erstreckt.

3. Prothese nach Anspruch 2, wobei das Ende des zweiten Abschnitts eine sich verjüngende Spitze aufweist.

4. Prothese nach einem der Ansprüche 2 bis 3, wobei sich der zweite Krümmungsradius vom ersten Krümmungsradius entlang einem Segment des zweiten Abschnitts zwischen dem Außenrand (2) und seinem Ende unterscheidet.

5. Prothese nach einem der vorhergehenden Ansprüche, wobei der erste Abschnitt (1) einen ersten unteren Rand (3) umfasst und der zweite Abschnitt (6) einen zweiten unteren Rand (7), und wobei der Körper einen dritten Abschnitt (9) umfasst, der mit dem ersten unteren Rand (3) verbunden ist, und einen vierten Abschnitt (13), der mit dem zweiten unteren Rand (7) verbunden ist, wobei der dritte und der vierte Abschnitt jeweils in einer im Wesentlichen sphärischen Gestalt konfiguriert sind.

6. Prothese nach einem der vorhergehenden Ansprüche, wobei der zweite Abschnitt einen Flächeninhalt und der Körper einen Gesamtflächeninhalt aufweist, wobei die Prothese in einem Verhältnis des Flächeninhalts des zweiten Abschnitts zum Gesamtflächeninhalt konfiguriert ist, welches in einem Bereich von etwa 0,25 bis etwa 0,40 liegt.

7. Prothese nach Anspruch 6, wobei der Gesamtflächeninhalt größer als etwa 40.000 mm² ist.

8. Prothese nach einem der Ansprüche 5 bis 7, wobei der Körper eine dauerhafte Vertiefung zwischen dem dritten Abschnitt (9) und dem vierten Abschnitt (13) umfasst, wobei die Vertiefung ausgebildet und angeordnet ist, um nahe den externen Iliac-Arterien angeordnet zu werden, wenn die Prothese an der Wand positioniert wird, um einen Leistenbruch zu reparieren.

9. Prothese nach Anspruch 8, wobei der Körper einen Scheitelpunkt (4) umfasst und sich die Vertiefung von einem Umfangsrand des Körpers einwärts zum Scheitelpunkt hin erstreckt.

10. Prothese nach einem der Ansprüche 5 bis 9, wobei der dritte Abschnitt (9) einen dritten Krümmungsradius aufweist und der vierte Abschnitt (13) einen vierten Krümmungsradius, der im Wesentlichen gleich dem dritten Krümmungsradius ist.

11. Prothese nach Anspruch 10, wobei der dritte Krümmungsradius etwa 35 mm beträgt.

12. Prothese nach einem der Ansprüche 5 bis 11, wobei der dritte und der vierte Abschnitt (9, 13) jeweils einen gekrümmten unteren Rand (12, 14) aufweisen.

13. Prothese nach einem der vorhergehenden Ansprüche, wobei der Körper einen im Wesentlichen D-förmigen Umfangsrand aufweist.

14. Prothese nach einem der vorhergehenden Ansprüche, wobei der erste Krümmungsradius etwa 120 mm beträgt.

15. Prothese nach einem der vorhergehenden Ansprüche, wobei der Körper aus prothetischem Material chirurgisches Netz umfasst.

16. Prothese nach einem der vorhergehenden Ansprüche, wobei der Körper einen Umfangsrand aufweist, der so konfiguriert ist, dass der Körper, nachdem dieser verformt wurde, wieder die konturierte Gestalt annehmen kann.

## Revendications

1. Une prothèse implantable destinée à la réparation d'un défaut dans une paroi musculaire ou tissulaire, cette prothèse comprenant un corps en matériau prothétique présentant une forme préformée tridimensionnelle qui prend indépendamment une forme arrondie adaptée en vue de se conformer à la paroi, ce corps incluant une première portion (1) ayant une configuration présentant une forme pratiquement sphérique et une seconde portion (6) reliée à la première portion le long d'un bord extérieur (2) de celle-ci, la seconde portion ayant une forme distincte de la première portion, la première portion ayant un premier rayon de courbure, **caractérisée en ce que** la seconde portion présente un second rayon de courbure qui est pratiquement égal au premier rayon de courbure le long du bord extérieur (2) dans une direction perpendiculaire au bord extérieur (2).

2. La prothèse selon la revendication 1, dans laquelle la seconde portion se termine par une extrémité opposée au bord extérieur et présente une forme arrondie s'étendant depuis le bord extérieur jusqu'à l'extrémité dans une direction perpendiculaire au bord extérieur (2).

3. La prothèse selon la revendication 2, dans laquelle l'extrémité de la seconde portion présente un bout pointu.

4. La prothèse selon l'une quelconque des revendications 2 et 3, dans laquelle le second rayon de courbure diffère du premier rayon de courbure le long d'un segment de la seconde portion située entre le bord extérieur 2 et l'extrémité de celle-ci.

5. La prothèse selon l'une quelconque des revendications précédentes, dans laquelle la première portion (1) comprend un premier bord inférieur (3) et la seconde portion (6) présente un second bord inférieur (7), le corps comportant une troisième portion (9) réunie au premier bord inférieur (3) et une quatrième portion (13) reliée au second bord inférieur (7), chacune des troisième et quatrième portions ayant une configuration de forme pratiquement sphérique.

6. La prothèse selon l'une quelconque des revendications précédentes, dans laquelle la seconde portion présente une superficie, et le corps présente une superficie totale, la prothèse ayant une configuration telle que le rapport de la superficie de la seconde portion à la superficie totale est de l'ordre d'environ 0,25 à environ 0,40.

7. La prothèse selon la revendication 6, dans laquelle la superficie totale est supérieure à environ 40 000 mm².

8. La prothèse selon l'une quelconque des revendications 5 à 7, dans laquelle le corps comprend une dépression permanente entre la troisième portion (9) et la quatrième portion (13), la dépression étant construite et agencée de manière à être située à proximité des vaisseaux iliaques externes quand la prothèse est mise en place sur la paroi, de manière à réparer une hernie inguinale.

9. La prothèse selon la revendication 8, dans laquelle le corps présente un sommet (4), la dépression s'étendant vers l'intérieur depuis un bord périphérique du corps vers ledit sommet.

10. La prothèse selon l'une quelconque des revendications 5 à 9, dans laquelle la troisième portion (9) présente un troisième rayon de courbure et la quatrième portion (13) présente un quatrième rayon de courbure qui est pratiquement égal au troisième rayon de courbure.

11. La prothèse selon la revendication 10, dans laquelle le troisième rayon de courbure est approximativement 35 mm.

12. La prothèse selon l'une quelconque des revendications 5 à 11, chacune des troisième et quatrième portions (9, 13) présente un bord inférieur arrondi (12, 14).

13. La prothèse selon l'une quelconque des revendications précédentes, dans laquelle le corps présente un bord périphérique de forme générale en D.

14. La prothèse selon l'une quelconque des revendications précédentes, dans laquelle le premier rayon de courbure est d'approximativement 120 mm.

15. La prothèse selon l'une quelconque des revendications précédentes, dans laquelle le corps en matériau prothétique est constitué par un filet chirurgical.

16. La prothèse selon l'une quelconque des revendications précédentes, dans laquelle le corps présente un bord périphérique dont la configuration permet au corps de reprendre sa forme après avoir été déformé.
